# EUROPEAN PATENT APPLICATION

(11) **EP 3 348 300 A1**
(43) Date of publication of application: **18.07.2018**
(21) Application number: 17169171.0
(22) Date of filing: 03.05.2017
(51) Int. Cl.: A61M 16/10, A61M 16/06, A61M 16/20, A61M 16/00, A61M 16/04, A61H 9/00, A62B 25/00

(54) **ELECTRONIC CONTROL CIRCUIT FOR ALLEVIATING ALTITUDE SICKNESS AND OXYGEN SUPPLY DEVICE**

(30) Priority: 13.01.2017 CN 201710023668
(71) Applicant: Cooling Electronics Co., Limited, Hong Kong (HK)
(72) Inventor: Lin, Mingtao, Hong Kong (HK)
(74) Representative: Petraz, Gilberto Luigi

(57) **Abstract**

Discloses are an electronic control circuit for alleviating altitude sickness and an oxygen supply device. The electronic control circuit includes an oxygen sensor (21) installed in an oxygen supply channel, a breathing frequency sensor (22) installed to the front of a head of a human body, and a microcontroller (23) coupled to a control button (24), a flow proportion electromagnet (25), a bypass electromagnet (26) and an air pump motor (27). The oxygen sensor and breathing frequency sensor detect analog signal and transmit the signal to the microcontroller after an analog-to-digital conversion, and an ON/OFF signal of the control button is transmitted to the microcontroller. The microcontroller has a flow control end coupled to a flow proportion electromagnet, a bypass control end coupled to a bypass electromagnet, and a pneumatic control end coupled to an air pump motor, and further includes a vibrating element (239) electrically coupled to the microcontroller. When the present invention detects a user's shortness of breathing or rapid breathing, the flow control valve, air pump and bypass circuit valve are turned on to supply oxygen with an appropriate oxygen concentration to users to alleviate altitude sickness.

## Description

### FIELD OF INVENTION

The present invention relates to an electronic control circuit, in particular to the electronic control circuit and device for supplying oxygen.

### BACKGROUND OF INVENTION

### 1. Description of the Related Art

As the standard of living improves, more and more people travel to plateaus and highlands. However, people living in plain areas once entering into the highland areas may suffer a severe altitude sickness. Most believe that altitude sickness is related to rarefied air and oxygen deficiency.

To alleviate the altitude sickness, people may buy a portable oxygen bottle and inhale oxygen through the mouth directly. Such portable oxygen bottle is not just inconvenient to use only, but also has the drawbacks of unable to control the consumption of oxygen, know the required concentration of oxygen, and when to resupply oxygen. Obviously, it is necessary to have an electronic control circuit to control and achieve the effect of resupplying oxygen appropriately and scientifically.

### 2. Summary of the Invention

Therefore, it is a primary objective of the present invention to overcome the drawbacks of the prior art by providing an electronic control circuit and an oxygen supply device for alleviating altitude sickness of the invention.

To achieve the aforementioned and other objectives, the present invention provides an electronic control circuit for alleviating altitude sickness comprising: a breathing frequency sensor, installed at the front of a head of a human body; and a microcontroller, coupled to a control button, a flow proportion electromagnet, a bypass electromagnet and an air pump motor; and the oxygen sensor and breathing frequency sensor detecting an analog signal and transmitting the signal to the microcontroller after an analog-to-digital conversion, and an ON/OFF signal of the control button being transmitted to the microcontroller, and the microcontroller having a flow control end coupled to a flow proportion electromagnet, a bypass end coupled to a bypass electromagnet, and a pneumatic control end coupled to an air pump motor, and further including a vibrating element electrically coupled to the microcontroller, and the microcontroller outputting an inverse proportional signal to the flow proportion electromagnet to supply a stable amount of oxygen according to an oxygen concentration sensing signal of the oxygen sensor, wherein if the oxygen concentration sensing signal of the oxygen sensor is lower than a predetermined lower oxygen concentration limit, the microcontroller will output a control signal to the air pump motor and bypass electromagnet, and the bypass electromagnet will be operated to turn off the bypass solenoid valve, and the air pump motor being will be on to increase an oxygen supply speed, and a maximum control signal will be outputted to the flow proportion electromagnet to increase the amount of oxygen supply; and if a sensing signal of the breathing frequency sensor exceeds a predetermined emergency setting, the microcontroller will output a control signal to the vibrating element, and the vibrating element will be turned on to produce a massage action, and the microcontroller will control the connection or disconnection of the bypass electromagnet according to the waveform of the sensing signal of the breathing frequency sensor, so that oxygen will not be outputted when a user exhales, and oxygen will be outputted when the user inhales.

Wherein, the microcontroller is coupled to a communication module, and the vibrating element is an electromagnetic armature vibrator.

Wherein, the communication module is a WiFi module for mobile phone communications, and the vibrating element further includes a heating plate for increasing the temperature of the vibrator.

Wherein, the electronic control circuit for alleviating altitude sickness further comprises a display screen for displaying an oxygen supply parameter, and the display screen is coupled to the microcontroller, and the oxygen supply channel further includes a heating element coupled to the microcontroller and provided for increasing the temperature of oxygen.

To achieve the aforementioned and other objectives, the present invention provides an oxygen supply device for alleviating altitude sickness, comprising: a casing, an oxygen bottle installed in the casing, an air pump coupled to a pipeline of the oxygen bottle, a bypass solenoid valve installed parallel to a pipeline of the air pump, and the air pump having an output end coupled to a flow control valve, and an outlet of the flow control valve being coupled to an air pipe interface disposed on a surface of the casing, and the oxygen supply device further comprising an air outlet pipe coupled to a pipeline of the air pipe interface, and the air outlet pipe having an end coupled to an air duct for coupling the pipeline of the casing and the other having an air outlet and a breathing frequency sensor, and the oxygen supply device further comprising a headset bracket for mounting the air outlet pipe, and the electronic control circuit of claim 1, and the oxygen sensor being installed at an outlet of the air pump, and the microcontroller being installed in the casing, and the control button being installed on a surface of the casing, and the flow proportion electromagnet being installed in the flow control valve, and the bypass electromagnet being installed in the bypass solenoid valve, and the air pump motor and the air pump being transmitted and coupled to each other, and the headset bracket further having a hat member or a headband coupled to the headset bracket and disposed around a user's head, and the vibrating element being disposed at the front of the hat member or headband and configured to be corresponsive to the user's forehead.

Wherein, the casing has a belt passing hole formed on an outer surface of the casing for passing a belt.

Wherein, the casing includes an accommodating cavity for placing a mobile phone.

Wherein, the casing includes a battery coupled to the microcontroller, and the battery is a rechargeable battery.

Wherein, the air outlet pipe has an outer end coupled to a flat air exchange pipe disposable in a user's mouth, and the flat air exchange pipe is a tubular structure penetrating through the front to the rear, and the air outlet pipe has an outer end coupled to a side of the flat air exchange pipe and intersected at an angle from 15 degrees to 45 degrees.

Wherein, the air outlet pipe has a U-shaped tube disposed at an outer end of the air outlet pipe and connectable to both of a user's nasal cavities, and the middle of the U-shaped tube is coupled and communicated with the air outlet pipe, and each of both ends of the U-shaped tube has an air outlet.

Compared with the prior art, the present invention has the following advantages and effects: The present invention collects the information of a user's breathing condition according to the breathing frequency sensor and coverts and processes the information by the microcontroller into the control signal of the flow control valve, the air pump, and the bypass circuit valve. Whenever a poor or rapid breathing condition of the user is detected, the flow control valve, the air pump and the bypass circuit valve will be turned on automatically to supply oxygen with an appropriate concentration to the user, so as to alleviate the user's altitude sickness that may occur later. The oxygen may be supplied intermittently according to the cycle of inhaling and exhaling movements to reduce the consumption of oxygen. The casing further has an accommodating cavity to facilitate the user to put a mobile phone, and includes a battery, so that the casing may serve as a power bank.

### BRIEF DESCRIPTION OF THE DRAWINGS

FIG. 1 is a schematic circuit block diagram of an electronic control circuit for alleviating altitude sickness in accordance with the present invention;
FIG. 2 is a schematic view of an oxygen supply device for alleviating altitude sickness in accordance with a preferred embodiment of the present invention; and
FIG. 3 is a schematic view of an air outlet pipe and a flat air exchange pipe of an oxygen supply device for alleviating altitude sickness in accordance with a preferred embodiment of the present invention.

### DESCRIPTION OF THE PREFERRED EMBODIMENTS

To make it easier for our examiner to understand the objective of the invention, its structure, innovative features, and performance, we use a preferred embodiment together with the attached drawings for the detailed description of the invention. Exemplary embodiments are illustrated in referenced figures of the drawings. It is intended that the embodiments and figures disclosed herein are to be considered illustrative rather than restrictive.

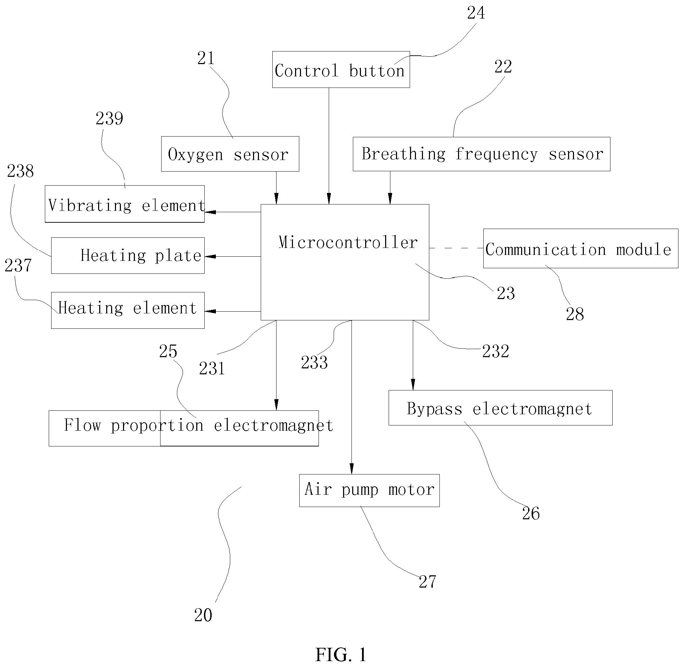

With reference to FIG. 1 for an electronic control circuit 20 for alleviating altitude sickness of the present invention, the electronic control circuit 20 comprises: an oxygen sensor 21 installed in an oxygen supply channel; a breathing frequency sensor 22 (or substituted by an airflow sensor for detecting a user's breathing frequency through the frequency of the change of airflow) installed at the front of a forehead of a human body; and a microcontroller 23 coupled to a control button 24, a flow proportion electromagnet 25, a bypass electromagnet 26 and an air pump motor 27, wherein the oxygen sensor 21 and the breathing frequency sensor 22 detect an analog signal and transmit the signal to the microcontroller 23 after an analog-to-digital conversion, an ON/OFF signal of the control button 24 is transmitted to the microcontroller 23, and the microcontroller 23 has a flow control end 231 coupled to the flow proportion electromagnet 25, a bypass control end 232 coupled to the bypass electromagnet 26, and a pneumatic control end 233 coupled to the air pump motor 27. The microcontroller 23 is further coupled to a communication module 28, and the communication module 28 of this preferred embodiment is a WiFi module for mobile phone communications and capable of transmitting data (such as the frequency of oxygen supply, and the concentration and time of oxygen supply each time) to a mobile phone, and then the mobile phone performs statistics of the data and provides these information as a reference for the users. Since the human body is oxygen deficient in highlands, the discomfort of altitude sickness may be alleviated by massage in addition to oxygen supplement. Therefore, the present invention may further comprise a vibrating element 239 electrically coupled to the microcontroller 23. The microcontroller 23 compares the sensing signal of the oxygen sensor 21, and outputs a control signal to the vibrating element 239 if the sensing signal is below a predetermined standard value. It is noteworthy that the control signal may be processed simultaneously or separately with the control signal for oxygen supply. For example, these control signals are processed once for the oxygen supply control and once for the vibration, and these signals are processed alternately and repeatedly. The vibrating element 239 is turned on by the control signal to produce a massage action. Wherein, the vibrating element is an electromagnetic armature vibrator (whose amplitude and frequency may be controlled by the microcontroller to provide a user-friendly control or using a motor belt with an eccentric wheel, so that the frequency but not the amplitude may change easily).

Specifically, the control is described as follows: In a general oxygen supply, the pressure in the oxygen bottle and the concentration of oxygen drop gradually during the process of its use, so that the microcontroller outputs an inverse proportion signal to the flow proportion electromagnet according to the oxygen concentration sensing signal of the oxygen sensor to supply a stable amount of oxygen.

In the situation of having a low oxygen supply level, the oxygen concentration sensing signal of the oxygen sensor is lower than a predetermined lower oxygen concentration limit (indicating that the oxygen content drops and reaches the lower limit), so that the microcontroller will output a control signal to the air pump motor and the bypass electromagnet to turn on the bypass electromagnet, turn off the bypass solenoid valve, turn on the air pump motor, and increase the oxygen supply speed, and a maximum control signal will be outputted to the flow proportion electromagnet to increase the amount of oxygen supply.

In an emergency situation requiring an assisting function, the sensing signal of the breathing frequency sensor exceeds the range of emergency settings, so that the microcontroller will output a control signal to the vibrating element to turn on the vibrating element and produce a massage action, so as to alleviate the discomfort of the altitude sickness.

To save the consumption of oxygen, no oxygen is outputted when the user exhales , and the oxygen is outputted only when the user inhales, so that the microcontroller will control the connection/disconnection of the bypass electromagnet according to the waveform of the sensing signal of the breathing frequency sensor, so as to achieve the aforementioned effect.

In this preferred embodiment, the vibrating element 239 further includes a heating plate 238 for increasing the temperature of the vibrator to provide a heated massage effect. The oxygen supply channel further includes a heating element 237 installed therein and coupled to the microcontroller 23 for increasing the temperature of oxygen, so as to fit the user better, particularly for the highland activities during winter.

Wherein, the microcontroller may be formed by a common single-chip device or a small PLC controller (such as Siemens Micro PLC controller), or any electronic components adopting a switch tube.

In another preferred embodiment, the invention further comprises a display screen for displaying an oxygen supply parameter, and the display screen may be a digital tube display screen.

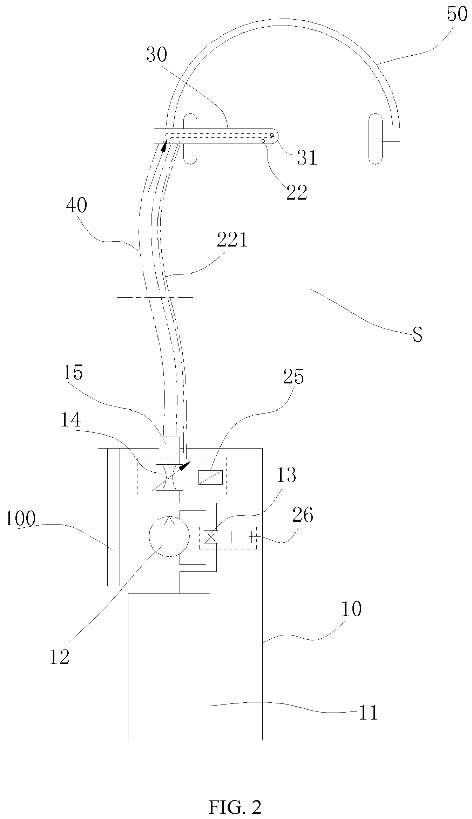

With reference to FIG. 2 for an oxygen supply device S for alleviating altitude sickness in accordance with a preferred embodiment of the present invention, the oxygen supply device comprises: a casing 10 having an oxygen bottle 11 installed in the casing 10, and air pump 12 installed in the casing 10 and coupled to a pipeline of the oxygen bottle 11, and a bypass solenoid valve 13 installed parallel to a pipeline of the air pump 12; and an output end of the air pump 12 is coupled to a flow control valve 14, and the outlet of the flow control valve 14 is coupled to an air pipe interface 15 on a surface of the casing 10. The invention further comprises an air outlet pipe 30 coupled to a pipeline of the air pipe interface 15, and the air outlet pipe 30 has an end coupled to an air duct 40 for connecting the pipeline of the casing and an air outlet 31 and a breathing frequency sensor 22 (connected by means of a conductive wire 221 and the microcontroller installed in the casing) installed a the other end of the air outlet pipe 30. The invention further comprises a headset bracket 50 for fixing the air outlet pipe 30, and the aforementioned electronic control circuit (not labeled in the figure, and whose main circuit devices are installed in the casing), and the oxygen sensor 21 is installed at the outlet of the air pump 12, and the microcontroller 23 is installed in the casing 10, and the control button 24 is installed to a surface of the casing 10, and the flow proportion electromagnet 25 is installed in the flow control valve 14 for controlling the flow control valve 14, and the bypass electromagnet 26 is installed in the bypass solenoid valve 13 for controlling the bypass solenoid valve 13, and the air pump motor 27 and the air pump 12 are transmitted and coupled to each other. A surface of the casing 10 further has a belt passing hole (not shown in the figure) for passing a belt, so that the user may carry the oxygen supply device at a user's back. To facilitate the user to place a mobile phone, the casing 10 further has an accommodating cavity 100 for placing the mobile phone.

Wherein, the headset bracket 50 is coupled to a hat member or headband (not shown in the figure) disposed around a user's head, and the vibrating element is disposed at the front of the hat member or headband and configured to be corresponsive to the forehead of a user to achieve the effect of massaging the forehead of the user.

In another preferred embodiment,

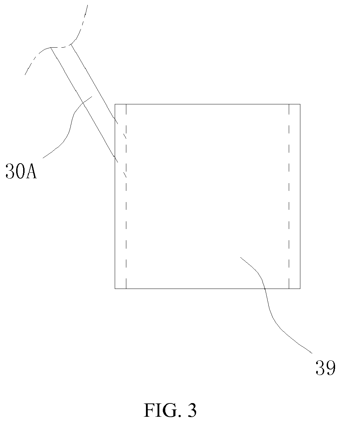

the air outlet pipe 30A has an outer end coupled to a flat air exchange pipe 39 (as shown in FIG. 3) disposable in a user's mouth, and the flat air exchange pipe 39 is a tubular structure penetrating through the front to the rear, and the air outlet pipe has an outer end coupled to a side of the flat air exchange pipe and intersected at an angle from 15 degrees to 45 degrees. Such structure allows the user to hold the flat air exchange pipe by the mouth for a long time, and the flat pipe fits the requirement of breathing freely, and the pipe is tilted to an angle from 15 to 45 degrees, so that the oxygen is entered towards the user's mouth without causing unnecessary waste of oxygen.

In another preferred embodiment, the air outlet pipe has a U-shaped tube disposed at an outer end of the air outlet pipe and connectable to both of a user's nasal cavities, and the middle of the U-shaped tube is coupled and communicated with the air outlet pipe, and each of both ends of the U-shaped tube has an air outlet. Therefore, such structure allows the air outlet to be placed in the user's nasal cavity and prevents any unnecessary waste of oxygen.

In another preferred embodiment, the casing further has a battery installed therein and coupled to the microcontroller, wherein the battery is a rechargeable battery.

In summation of the description above, the present invention collects the information of a user's breathing condition according to the breathing frequency sensor and coverts and processes the information by the microcontroller into the control signal of the flow control valve, the air pump, and the bypass circuit valve. Whenever a poor or rapid breathing condition of the user is detected, the flow control valve, the air pump and the bypass circuit valve will be turned on automatically to supply oxygen with an appropriate concentration to the user, so as to alleviate the user's altitude sickness that may occur later. The oxygen may be supplied intermittently according to the cycle of inhaling and exhaling movements to reduce the consumption of oxygen. The casing further has an accommodating cavity to facilitate the user to put a mobile phone, and includes a battery, so that the casing may serve as a power bank.

While the invention has been described by means of specific embodiments, numerous modifications and variations could be made thereto by those skilled in the art without departing from the scope and spirit of the invention set forth in the claims.

## Claims

1. An electronic control circuit for alleviating altitude sickness, comprising:
an oxygen sensor, installed in an oxygen supply channel; a breathing frequency sensor, installed at the front of a head of a human body; and a microcontroller, coupled to a control button, a flow proportion electromagnet, a bypass electromagnet and an air pump motor; and the oxygen sensor and breathing frequency sensor detecting an analog signal and transmitting the signal to the microcontroller after an analog-to-digital conversion, and an ON/OFF signal of the control button being transmitted to the microcontroller, and the microcontroller having a flow control end coupled to a flow proportion electromagnet, a bypass end coupled to a bypass electromagnet, and a pneumatic control end coupled to an air pump motor, and further including a vibrating element electrically coupled to the microcontroller, and the microcontroller outputting an inverse proportional signal to the flow proportion electromagnet to supply a stable amount of oxygen according to an oxygen concentration sensing signal of the oxygen sensor, wherein if the oxygen concentration sensing signal of the oxygen sensor is lower than a predetermined lower oxygen concentration limit, the microcontroller will output a control signal to the air pump motor and bypass electromagnet, and the bypass electromagnet will be operated to turn off the bypass solenoid valve, and the air pump motor being will be on to increase an oxygen supply speed, and a maximum control signal will be outputted to the flow proportion electromagnet to increase the amount of oxygen supply; and if a sensing signal of the breathing frequency sensor exceeds a predetermined emergency setting, the microcontroller will output a control signal to the vibrating element, and the vibrating element will be turned on to produce a massage action, and the microcontroller will control the connection or disconnection of the bypass electromagnet according to the waveform of the sensing signal of the breathing frequency sensor, so that oxygen will not be outputted when a user exhales, and oxygen will be outputted when the user inhales.

2. The electronic control circuit for alleviating altitude sickness according to claim 1, wherein the microcontroller is coupled to a communication module, and the vibrating element is an electromagnetic armature vibrator.

3. The electronic control circuit for alleviating altitude sickness according to claim 2, wherein the communication module is a WiFi module for mobile phone communications, and the vibrating element further includes a heating plate for increasing the temperature of the vibrator.

4. The electronic control circuit for alleviating altitude sickness according to claim 1, further comprising a display screen for displaying an oxygen supply parameter, and the display screen being coupled to the microcontroller, and the oxygen supply channel further includes a heating element coupled to the microcontroller and provided for increasing the temperature of oxygen.

5. An oxygen supply device for alleviating altitude sickness, comprising a casing, an oxygen bottle installed in the casing, an air pump coupled to a pipeline of the oxygen bottle, a bypass solenoid valve installed parallel to a pipeline of the air pump, and the air pump having an output end coupled to a flow control valve, and an outlet of the flow control valve being coupled to an air pipe interface disposed on a surface of the casing, and the oxygen supply device further comprising an air outlet pipe coupled to a pipeline of the air pipe interface, and the air outlet pipe having an end coupled to an air duct for coupling the pipeline of the casing and the other having an air outlet and a breathing frequency sensor, and the oxygen supply device further comprising a headset bracket for mounting the air outlet pipe, and the electronic control circuit of claim 1, and the oxygen sensor being installed at an outlet of the air pump, and the microcontroller being installed in the casing, and the control button being installed on a surface of the casing, and the flow proportion electromagnet being installed in the flow control valve, and the bypass electromagnet being installed in the bypass solenoid valve, and the air pump motor and the air pump being transmitted and coupled to each other, and the headset bracket further having a hat member or a headband coupled to the headset bracket and disposed around a user's head, and the vibrating element being disposed at the front of the hat member or headband and configured to be corresponsive to the user's forehead.

6. The oxygen supply device for alleviating altitude sickness according to claim 5, wherein the casing has a belt passing hole formed on an outer surface of the casing for passing a belt.

7. The oxygen supply device for alleviating altitude sickness according to claim 6, wherein the casing includes an accommodating cavity for placing a mobile phone.

8. The oxygen supply device for alleviating altitude sickness according to claim 5, wherein the casing includes a battery coupled to the microcontroller, and the battery is a rechargeable battery.

9. The oxygen supply device for alleviating altitude sickness according to claim 5, wherein the air outlet pipe has an outer end coupled to a flat air exchange pipe disposable in a user's mouth, and the flat air exchange pipe is a tubular structure penetrating through the front to the rear, and the air outlet pipe has an outer end coupled to a side of the flat air exchange pipe and intersected at an angle from 15 degrees to 45 degrees.

10. The oxygen supply device for alleviating altitude sickness according to claim 5, wherein the air outlet pipe has a U-shaped tube disposed at an outer end of the air outlet pipe and connectable to both of a user's nasal cavities, and the middle of the U-shaped tube is coupled and communicated with the air outlet pipe, and each of both ends of the U-shaped tube has an air outlet.
